# EUROPEAN PATENT SPECIFICATION

(11) **EP 3 781 013 B1**
(45) Date of publication and mention of the grant of the patent: **29.05.2024**
(21) Application number: 19719480.6
(22) Date of filing: 18.04.2019
(51) Int. Cl.: A61B 3/00, A61F 9/008

(54) **CUSTOMIZED ABLATION TO CORRECT VISUAL AMETROPIA**
KUNDENSPEZIFISCHE ABLATION ZUR KORREKTUR VON FEHLSICHTIGKEIT
ABLATION PERSONNALISÉE POUR CORRIGER UNE AMÉTROPIE VISUELLE

(30) Priority: 20.04.2018 DE 102018206163
(43) Date of publication of application: 24.02.2021
(73) Proprietor: IVIS TECHNOLOGIES S.R.L., 74100 Taranto (IT)
(72) Inventor: D' IPPOLITO, Giuseppe, 74100 Taranto (IT)
(74) Representative: Bardehle Pagenberg Partnerschaft mbB Patentanwälte Rechtsanwälte
(86) International application number: PCT/EP2019/060147
(87) International publication number: WO 2019/202104

(56) References cited:
- US-A1- 2004 024 389
- US-A1- 2007 161 972
- US-A1- 2009 281 529
- US-A1- 2015 131 054

## Description

### 1. Technical field:

The present invention relates to customized ablation to correct visual ametropia, and in particular to determining a customized volume of corneal tissue to be ablated, and a corresponding apparatus.

### 2. Description of the prior art:

Corneal refractive surgery has significantly evolved over the last twenty years leading to customized ablation, wherein ablation profiles are adapted to the specific needs of individual patients to correct their visual ametropia.

In treating the cornea of an eye to correct visual ametropia, usually a refractive error is first determined (e.g., refractive aberration information). A modified curvature of the anterior corneal surface is then calculated to correct the patient's vision, and the cornea is treated by means of a laser to obtain the modified profile. Thereby, a desired or target profile shall be obtained, which in turn has a desired refractive behavior that corrects the visual ametropia.

In the prior art, several methods are known for determining the target profile of the anterior corneal surface. In an example, it may be subjectively determined by the treating doctor, i.e., the treating doctor may determine the target profile such that it fulfills clinical requirements based on the doctor's clinical experience. WO 98/42291 relates to such an approach. Based on an available resolution, a set of points on the cornea of the eye is determined together with a position of each point. A computer then determines, for each point out of the determined set of points, a distance between the actual surface of the cornea and a predetermined reference surface. The predetermined reference surface may be determined based on clinical experience and or by ray-tracing. The determined distances then define the profile for corneal tissue ablation.

In another example, diopter correction values may be determined for various points on the cornea of the patient. These diopter correction values may be determined by searching diopter values which optimize a focalization at foveal level of a light ray impinging on the cornea as generated by a light source arranged at infinite distance (EP 1352 623). The set of diopter correction values then defines the profile for corneal tissue ablation.

US 2015/131054 A1 proposes a process and an apparatus for determining optical aberrations of an eye with its optical system including the cornea and the lens. The process includes the reconstructing of wavefront aberrations of the eye as a deviation of the wavefront, determined by the optical system of the eye with a process of aberrometry, with respect to an ideal planar wavefront generated by an aberration-free eye model. A measured ocular length is employed for the aberration-free eye model. However, the known methods take into account the structure of the eye only to a limited extent. Correspondingly, the current methods to determine the target profile of the cornea are not always optimal in terms of improvement of the quality of vision. Even more so, the current methods may lead to an ablation of an amount of corneal tissue which may be significantly higher than required.

Therefore, there is a need for improving the determination of the target profile of the cornea such that corneal tissue ablation may be minimized and/or such that the quality of vision is further enhanced.

### 3. Summary of the invention:

According to an aspect, the above need is at least partly met by an apparatus according to claim 1, and by a corresponding computer program and a corresponding method according to claims 16 and 27, respectively.

In an example, an apparatus is provided for visual ametropia correction. The apparatus comprises means for obtaining topographic information of an anterior surface and topographic information of a posterior surface of a cornea of an eye. The apparatus further comprises means for obtaining a desired focusing point within the eye. The apparatus also comprises means for determining correction information relating to the anterior surface of the cornea, such as to optimize focusing onto the desired focusing point, based on the topographic information of the anterior surface and of the posterior surface of the cornea. The correction information may comprise a correction that needs to be applied to the anterior corneal surface, e.g., such that light rays refracted by the corrected anterior corneal surface and by the posterior surface are optimally focused onto the desired focusing point.

The correction information may define a correction in the form of an ablation volume, an ablation profile and/or a target profile and/or a target slope of the anterior corneal surface, for example. The correction information may include values relating to individual points on the anterior surface of the cornea, e.g., a grid of points on the anterior surface of the cornea. The correction information may then be used to ablate a corresponding corneal volume.

Taking into account the topographic information of the posterior corneal surface - and not only the anterior corneal surface - in the determination of the correction information allows obtaining an ablation volume which better fits the needs of the individual patient and improves vision correction, since the impact of abnormalities and irregularities of the posterior surface may be taken into account. Moreover, it also may allow reducing the ablation volume.

An underlying idea of the present invention is that a major limitation of prior art approaches is caused by the fact that these neglect the refractive contribution of the posterior corneal surface. For example, in case of an irregular shape of the posterior corneal surface, a correction that is calculated solely based on the anterior corneal surface may not be satisfactory, since the (neglected) irregular posterior corneal shape may lead to distortions despite the applied correction. The correction is therefore not optimal. Since in the real eye, refraction occurs not only at the anterior corneal surface but also at the posterior corneal surface (due to a transition from the denser corneal stroma of the eye to the less dense aqueous humour of the eye), taking into account the specific patient's posterior corneal surface allows a better determination of the correction information. Thus, the vision correction of the individual patient may be improved. This is particularly the case, since posterior corneal surfaces may strongly vary from patient to patient, and may have irregular shapes (which may in general be described not only by linear or quadratic functions, but also by higher order functions, e.g., functions of third order and above, e.g. polynomials that may not have a shape that could be approximated by a sphere or an ellipse, etc.). Considering the specific topography (morphology or shape) of the posterior surface of the cornea when determining the correction information that optimizes focusing onto the desired focusing point, allows alleviating this issue. The refractive contribution of the posterior corneal surface of the individual patient is taken into account, which allows optimizing the correction and often minimizing the ablation volume necessary for the correction.

Topographic information of a surface of the cornea (e.g., a posterior and/or anterior surface) may include a profile (e.g., a plurality of coordinates defining one or more actual points on the surface of the cornea, a plurality of points approximating the surface of the cornea, or a function fitted to the surface of the cornea, etc.). The topographic information may for example be provided in the form of a number of points in an x-y-z coordinate system. Of course, other coordinate systems may be used. The correction information may then be determined based on the topographic information of the anterior and posterior corneal surfaces as well as the desired focusing point.

It is noted that the apparatus described above may not necessarily include means for determining or calculating the topographic information and/or the desired focusing point. The means for obtaining may be implemented by means for receiving the respective information from other devices or an operator of the apparatus. In such examples, the apparatus may use the received information directly for determining the correction information and/or the desired focusing point, and/or it may further process the received information. For example, the topographic information and/or the desired focusing point relating to a patient may be retrieved from a memory, a database, a server in a cloud, and/or from corresponding diagnostic devices, or these may simply be input by an operator of the apparatus, e.g., a surgeon.

In some examples, the correction information may comprise a target slope and/or a target profile of the anterior surface of the cornea. For example, the correction information may relate to one or more points (e.g., forming a grid) on the anterior surface of the cornea, wherein for each point a target slope or target thickness may be indicated, e.g. in form of a map or a profile of the target anterior corneal surface. The correction information may however also relate to values that are to be ablated, e.g. a target ablation volume and/or a target ablation thickness (for each point). Target ablation values may be obtained by intersecting the topographic information of the anterior corneal surface and, e.g., a target profile for the anterior corneal surface that optimizes vision.

In some examples, the means for determining may be adapted to perform, based on the topographic information of the anterior surface and on the topographic information of the posterior surface of the cornea, ray-tracing for at least one light ray passing through the cornea and being refracted by the anterior and posterior surfaces of the cornea. As known to the skilled person, refraction at an interface between two materials (e.g., air and corneal tissue, or corneal tissue and the aqueous humour of the eye) may be calculated, e.g., based on Snell's law and the refractive indices of the materials. Thus, based on the topographic information (e.g. a profile) of the corneal surface(s) and the refractive indices of the relevant materials (known to the skilled person), the refraction for light rays passing through the anterior and posterior surfaces of the cornea may be calculated.

Using the ray-tracing mentioned in the preceding paragraph, the correction information may be determined at least in part such as to optimize focusing of the traced ray onto the desired focusing point. In other words, the correction that needs to be applied to a certain point on the anterior surface of the cornea is determined, such that the light ray passing through that point is optimally focused onto the desired focusing point, as calculated based on the (corrected) anterior surface topography and based on the topographic information of the posterior surface.

In some examples, a region within a certain radius around a corneal apex (or a center of the anterior corneal surface) may be considered for ray-tracing. For example, a region on the anterior surface which is within a diameter corresponding to the diameter of the pupil of the eye, detected according to a predefined light environment, preferably, but not necessarily, obtained by a pupillometer, may be considered. For a regular or randomized grid of points within such a region, ray-tracing may then be performed, such that for each point, the corresponding correction information may be obtained, e.g. the target slope of the anterior surface at that point. By using an appropriate number points, correction information may be determined in the form of a map of the anterior corneal surface. In some example, a radius corresponding to the diameter of the pupil of the eye, detected according to a predefined light environment may be used (e.g. for far-field calculations), or an even smaller radius may be considered (e.g. for near-field calculations). Notably, in case of use of the pupil data detected by a pupillometer, it needs not to be part of the apparatus for that matter, but it would be sufficient if the apparatus is adapted to receive corresponding pupil data, e.g. from a storage device, or from the operator of the apparatus via a corresponding interface.

In some examples, the ray-tracing relates to at least one first light ray impinging on the anterior surface of the cornea in a manner parallel to an optical axis of the eye (forward ray-tracing). For example, forward ray-tracing may be performed for a plurality of first light rays, each first light ray impinging on a respective point of a grid of points within a selected region of the anterior surface of the cornea, as outlined in the preceding paragraph.

In the case of forward ray-tracing, the correction information relating to the anterior surface of the cornea may be determined at least in part such as to minimize a distance between the desired focusing point and an actual focusing point, as determined by the ray-tracing. The latter may be defined as an intersection of the at least one first light ray with an optical axis of the eye, after being refracted by the anterior and posterior corneal surfaces (as determined by the respective topographic information). For example, a target slope of the anterior corneal surface may be determined, such that the distance between the desired focusing point and the mentioned intersection is essentially zero. By doing this determination for several light rays, wherein each light ray impinges on a point of a grid of points on the anterior surface, the necessary correction information to be applied at that point may be determined, such that each light ray impinging on the respective point is optimally focused onto the desired focusing point. Thus, correction information, e.g. a target slope, for the entire grid of points may be obtained. A target profile of the anterior corneal surface may then be determined, e.g. by integrating the determined target slopes.

In some examples, additionally or alternatively to forward ray-tracing, the ray-tracing may relate to at least one second light ray emerging from the desired focusing point (reverse ray-tracing). For example, reverse ray-tracing may be performed for a plurality of second light rays, each second light ray exiting the cornea on a respective point on a grid of points within a selected region of the anterior surface of the cornea, after being refracted by the posterior and anterior corneal surfaces.

In the case of reverse ray-tracing, the correction information relating to the anterior surface of the cornea may be determined at least in part such as to minimize an angle between an optical axis of the eye and the at least one second light ray as it exits the cornea, after being refracted by the posterior and anterior corneal surfaces (as determined by the respective topographic information). For example, the correction information may be determined such that the angle between the ray-traced light ray exiting the cornea and the optical axis is essentially zero. Also in this manner, for a grid of points on the anterior surface of the cornea, the necessary correction information may be determined, such that, for each single point of the grid, the angle between the ray-traced light ray exiting the cornea and the optical axis is essentially zero.

The means for obtaining the desired focusing point is adapted to determine the desired focusing point based on the topographic information of the anterior surface and of the posterior surface of the cornea and based on refractive aberration information for the eye. Such desired focusing point is determined by ray-tracing. For example, from the topographic information of the anterior and posterior surfaces of the cornea, corresponding focusing points may be calculated for a set of light rays. Moreover, from the refractive aberration information, e.g. subjective refraction, it maybe calculated, e.g. for each light ray, how the corresponding focusing point needs to be shifted. For example, a set of light rays parallel to an optical axis of the eye, and refracted according to the correction information (e.g., by passing through a lens corresponding to the correction information, such that glasses would be imitated) may be traced through the cornea. In particular, based on the topographic information of the anterior and posterior corneal surfaces, it may be determined, at which coordinates each light ray intersects the optical axis. The desired focusing point may then be determined as a weighted average of these coordinates. In some examples, a region within a certain radius around the corneal apex (or a center of the anterior corneal surface) may be considered. For example, a region of the anterior surface which corresponds to the (photopic) diameter of the pupil of the eye, preferably obtained by a pupillometer, may be considered. Further, the set of light rays may correspond to a grid of points, as outlined with reference to the topographic information and/or the correction information. Thus, the desired focusing point may be optimally estimated, taking into account also the refractive contribution of the posterior corneal surface.

In other examples, the desired focusing point may simply be entered by the operator of the apparatus. For example, it may be selected by the operator to correspond to the position of the fovea or any other location on the patient's retina or it may be defined arbitrarily, depending on the specific refractive aberration the operator would like to correct.

The apparatus may comprise means for obtaining refractive aberration information for the eye. For example, it may comprise an interface, via which an operator of the apparatus may input the refractive aberration information, and/or via which the refractive aberration information may be obtained from a storage medium.

The refractive aberration information may comprise subjective refraction for the eye (such as one or more spherical diopter values, and/or one or more cylindrical diopter values and one or more cylinder axes, for example), e.g. as obtainable by a typical vision test. Thus, in some examples, the refractive aberration information comprises or consists of subjective refraction.

Additionally or alternatively, the apparatus may comprise means for obtaining refractive aberration information for the eye from a scanning laser refractometer (e.g. as disclosed by EP 1459 676) and/or a wave-front analyzer or a similar device. For example, the refractive aberration information may comprise one or more parameters, e.g. polynomial coefficients, obtainable from a wave-front analyzer. The scanning laser refractometer and/or the wave-front analyzer may for example be adapted to provide the refractive aberration information in the form of a map of refractive errors, e.g., for selected regions on the anterior surface of the cornea, as explained. For example, the refractive errors may be expressed as diopter values, coefficients for one or more polynomials, such as Zernike polynomials, etc. The apparatus may include a scanning laser refractometer and/or wave-front analyzer or simply may import, by means of any media, the refractive aberration information from a laser refractometer and/or a wave-front analyzer.

Based on the obtained correction information, e.g. based on a target profile of the anterior surface, one or more of the above steps may be repeated. For example, the obtained target profile may be used, together with the topographic information of the posterior corneal surface and the desired focusing point, to carry out a further iteration of determining correction information. For example, it may be verified, e.g., by ray-tracing as outlined above, whether - taking into account the target profile, instead of the topographic information of the anterior surface as is - indeed leads to a focusing of each ray onto the desired focusing point and/or an emergence of light rays from the desired focusing point out of the cornea parallel to the optical axis, or whether further correction is needed. Such iterative process may be beneficial, particularly such as to avoid a target profile which may include coarse steps, or which may be discontinuous.

In some examples, topographic information relating to one or more internal interfaces of the cornea may be taken into account in the same manner as described herein with respect to topographic information of the anterior and posterior surfaces. For example, topographic information regarding the surfaces of a corneal epithelium (a relatively thin tissue layer located at the anterior cornea) and/or a corneal stroma (a relatively thick and transparent middle layer of the cornea) may be used. This information may be obtained, for example, by means of a tomographer, similarly as outlined herein with respect to the anterior and posterior corneal surfaces. Moreover, it may be used for determining the correction information, e.g., by means of ray-tracing that also takes into account the refraction of light rays at the corresponding interfaces.

In an example, the apparatus may be adapted to obtain the topographic information of the anterior corneal surface and the posterior corneal surface of the cornea of the eye from a corneal tomographer. The tomographer may be based on light scattering techniques and/or ultrasonic techniques, etc. Additionally or alternatively, optical coherence tomography techniques may be used.

In a further example, the topographic information of the anterior corneal surface and the posterior corneal surface obtained from the corneal tomographer may be used for ray-tracing, as described herein. For example, the correction that needs to be applied to a certain point on the anterior surface of the cornea (obtained from the corneal tomographer) is determined, such that the light ray passing through that point is optimally focused onto a desired focusing point, as calculated based on the (corrected) anterior surface topography and based on the topographic information of the posterior surface (obtained from the corneal tomographer). In some examples, this may be done without any fitted parameters. In some example, only the topographic information of the anterior corneal surface and the posterior corneal surface (obtained from the corneal tomographer) may be used. In yet a further example, the desired focusing point may be determined by ray-tracing based on the topographic information of the anterior corneal surface and the posterior corneal surface obtained from the corneal tomographer. In some examples, this may be done without any fitted parameters. Moreover, determining the desired focusing point from ray-tracing may in some examples be based on topographic information of the anterior corneal surface and the posterior corneal surface only.

The apparatus may comprise the tomographer. The use of the corneal tomographer allows, e.g., in contrast to corneal topographers based on Placido rings, not only the determination of anterior corneal surface properties but also properties regarding the thickness of the cornea, and in particular the posterior surface. For example, the corneal tomographer may provide topographic information such as a profile of the anterior and the posterior surfaces of the cornea. It may also provide thickness information about the cornea. It may also provide more detailed information about the individual layers of the cornea, such as a corneal epithelium and/or a corneal stroma. In some examples, topographic information regarding the various layers of the cornea may also be taken into account when determining the correction information, as mentioned, which may allow an even more precise determination of the target anterior corneal surface to optimize vision.

In a further example, the apparatus may be adapted to obtain the topographic information, at least in part, from a pupillometer to determine a (photopic) diameter of a pupil of the eye. The apparatus may comprise such a pupillometer. The pupillometer may also be used to define a minimum region of the anterior corneal surface for which the visual ametropia may be corrected, the region having a diameter determined according to a predefined light environment. In some examples, the region may also be determined subjectively and received by the apparatus. Alternatively, the operator may preselect the region of the eye which is to be treated and may then, in a next step, refine the preselected region by using the information provided by the pupillometer, e.g., by using the pupil diameter. All in all, this allows to precisely limit the region of the cornea which is to be treated by the laser procedure. Consequently, the invasiveness of the surgical treatment may be reduced.

In a further example, the apparatus may also comprise means for correcting an aberration of the eye based on the determined correction information, preferably a laser to remove corneal tissue. For example, an excimer laser or a solid-state laser may be used to that end. In other examples, the apparatus may be adapted to determine the correction information such that it may be provided to a laser to remove corneal tissue, without the laser necessarily being part of the apparatus.

In some examples, the apparatus may be adapted to control the means for correcting with a closed-loop feedback. For example, the apparatus may comprise means for controlling a laser (or more generally: the means for correcting) based on updated topographic information of the anterior surface of the cornea. During ablation by the laser, the topographic information of the anterior surface of the cornea may be updated, e.g. in real-time, and the ablation may be controlled accordingly, e.g. in a closed-loop. For example, the ablation may be stopped if a sufficient agreement is reached between the updated topographic information and the correction information, e.g. a target profile of the anterior surface of the cornea, while it may be controlled to continue as long as such agreement has not been reached.

The apparatus may also be adapted to store the determined correction information, e.g., on a memory, in a database, or a server. The correction information may then be retrieved by the means for correcting the aberration of the eye, e.g. the laser, which may be part of the apparatus or which may be separately provided.

In an example, the corneal tomographer, the scanning laser refractometer and/or the wave-front analyzer, the laser, and optionally the pupillometer may be comprised by the apparatus such that they are an integral part of the apparatus. In such examples, the apparatus may comprise a processing and/or control unit (e.g. a computer) which interacts with the mentioned other parts of the apparatus. For example, the topographic information and/or the refractive aberration information may be transmitted to the processing and/or control unit, e.g. via one or more wires and/or wirelessly. For example, the correction information may similarly be transmitted from the processing and/or control unit to the laser. In another example, the various mentioned parts may not necessarily be an integral part of the apparatus, thereby providing a modular set-up. In this example, the apparatus (which may still comprise a processing unit) may be adapted to exchange the topographic information, the refractive aberration information, and/or correction information, e.g. via one or more wires and/or wirelessly. For example, the respective information may be wirelessly transmitted to and from the apparatus, respectively.

It is noted that the aspects relating to the determination of the desired focusing point may be combined with the further aspects of the present invention, but it may also be implemented independently from these further aspects. For example, a method and/or apparatus may be provided related to obtaining topographic information of an anterior surface and topographic information of a posterior surface of a cornea of an eye. Further, refractive aberration information for the eye may be obtained. A desired focus point may be determined based on the topographic information of the anterior surface and of the posterior surface of the cornea, and based on refractive aberration information, e.g. by ray-tracing.

A further embodiment of the present invention relates to a computer program comprising instructions for carrying out the steps and/or implementing the means described herein. For example, the computer program may be stored on a memory medium and it may cause a processor to implement the individual steps. The memory medium and/or the processor may be comprised by an apparatus according to the present invention. The apparatus of the present invention may generally comprise the computer program of the present invention.

A further embodiment of the present invention relates to a method which may comprise the step of obtaining topographic information of an anterior surface and topographic information of a posterior surface of a cornea of an eye. The method may further comprise the step of obtaining a desired focusing point within the eye. The method may further comprise determining correction information relating to the anterior surface of the cornea, such as to optimize focusing onto the desired focusing point, based on the topographic information of the anterior surface and of the posterior surface of the cornea. The method may optionally also comprise further steps, which are described herein with reference to an apparatus and/or a computer program. The method does not include the steps for actually correcting the aberration of the eye based on the determined correction information.

### 4. Brief description of the Figures:

Possible embodiments of the present invention will be described in more detail in the subsequent detailed description with reference to the following Figures:
- Fig. 1:: Example flow chart for customized corneal ablation according to an example of the present invention;
- Fig. 2a:: Schematic representation of an exemplary corneal cross-section including an anterior corneal surface of an eye and a target anterior corneal surface;
- Fig. 2b:: Top view of an example region of an anterior corneal surface which may be surgically treated comprising a monofocal refractive zone and a customized connecting zone.
- Fig. 2c:: Top view of an example region of the cornea which is to be surgically treated comprising a multifocal refractive zone subdivided into a near vision zone, an intermediate vision zone, and a far vision zone, and a customized connecting zone surrounding the multifocal zone.
- Fig. 3:: Schematic representation of an exemplary corneal cross-section comprising an anterior corneal surface, a posterior corneal surface, and slopes for various points on the anterior and the posterior corneal surfaces.
- Fig. 4a:: Example for determining a region of the anterior surface of the cornea relevant for vision based on the topographic information of the anterior and posterior corneal surfaces and based on a predefined, preferably photopic pupil diameter.
- Fig. 4b:: Example for determining a desired focusing point based on the topographic information of the anterior and posterior corneal surfaces, and based on refractive aberration information.
- Fig. 4c:: Example for determining a region of the anterior surface of the cornea for which correction information is to be calculated based on the topographic information of the anterior and posterior corneal surfaces, based on the desired focusing point and based on the diameter of the pupil of the eye, detected according to a predefined light environment.
- Fig. 5a:: First example for determining correction information based on the topographic information of the anterior and posterior corneal surfaces, and based on the desired focusing point by forward ray-tracing.
- Fig. 5b:: Second example for determining correction information based on the topographic information of the anterior and posterior corneal surfaces, and based on the desired focusing point by reverse ray-tracing.
- Fig. 6:: Exemplary cross-section of a cornea with a target profile of the anterior corneal surface.

### 5. Detailed description of possible embodiments:

Possible embodiments of the present invention will be described in the following. For brevity, only a few embodiments can be described. The skilled person will recognize that the specific features described with reference to these embodiments may be modified and combined differently and that individual features may also be omitted if they are not essential. The general explanations in the sections above will also be valid for the following more detailed explanations.

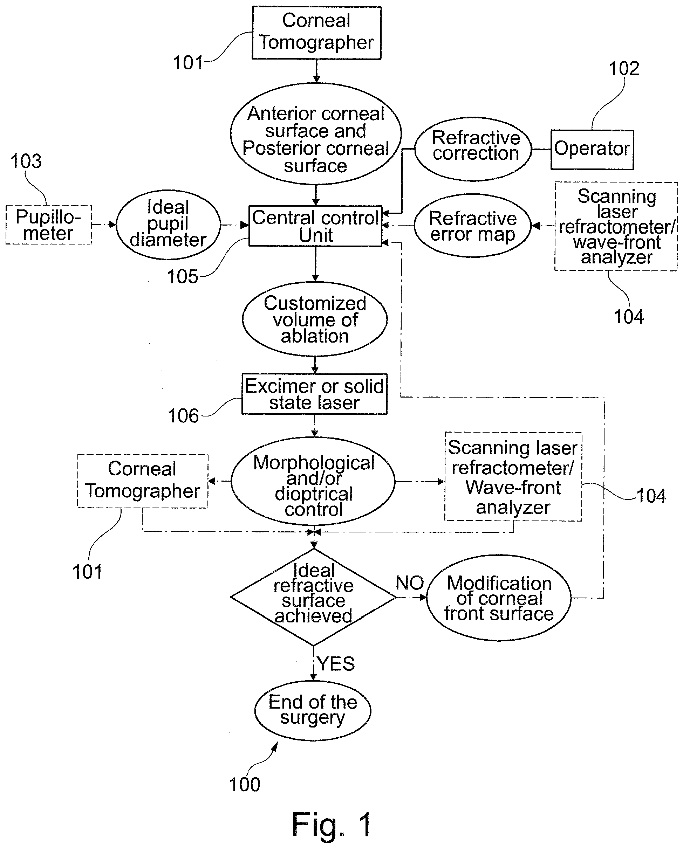

Fig. 1 shows an example flow chart 100 for customized corneal ablation by means of an apparatus according to the present invention.

In this example, the apparatus comprises a corneal tomographer 101, a control unit 105 (e.g., a computer), and an excimer or solid state laser 106. Reference numeral 102 indicates an operator, e.g., a treating doctor. In this example, the apparatus may optionally comprise a pupillometer 103, and/or a scanning laser refractometer and/or a wave-front analyzer 104.

The corneal tomographer 101 may be adapted to provide topographic information of the anterior corneal surface and the posterior corneal surface. The tomographer may be based on light scattering techniques, ultrasonic techniques, and/or optical coherence tomography, for example. The provided topographic information may comprise a profile, and possibly a slope profile and/or a curvature profile, of the anterior and the posterior surfaces of the cornea. It may also provide thickness information about the cornea and/or even more detailed information about the individual layers of the cornea, such as a corneal epithelium and/or a corneal stroma.

At reference number 102, refractive aberration information for the eye may be obtained by the apparatus from an operator, e.g., the treating doctor. This information may be refractive aberration information, which may, e.g., be subjectively be determined by the operator based on the operator's clinical experience. For example, correction information may include spherical dioptrical correction, cylindrical dioptrical correction and related axis, etc., as may be determined by a usual vision test or by a schiascopic examination or by an autorefractometer

Additionally or alternatively to the refractive aberration information obtained from the operator, such information may be obtained from a scanning laser refractometer and/or a wave-front analyzer 104. The scanning laser refractometer and/or the wave-front analyzer may provide refractive aberration information such as a refractive error map, e.g., for a cross-section of the cornea, and/or for a two-dimensional region of the surface of the cornea. For example, the refractive error may be expressed as angular errors or angular slopes or any other suitable format. The refractive error may relate to an error as a function of planar x and y (or any other) coordinates of the anterior corneal surface.

The pupillometer 103 may be used to determine, e.g., the photopic pupil diameter, e.g. the pupil diameter detected according to a predefined light environment. The photopic pupil diameter may be preferably but not mandatorily used to define the desired focusing point and the pupil diameter detected according to a predefined light environment may be preferably but not mandatorily used to limit the region of the cornea which is to be surgically treated. The regions of the cornea corresponding to the photopic pupil diameter and to the pupil diameter detected according to a predefined light environment are determined by means of a ray tracing process. This may allow, e.g., the operator to better refine the preselected region of the cornea for treatment. Moreover, it may be determined which region of the cornea should be used for determining a desired focusing point (e.g., by means of ray-tracing). For the above determinations, it may be useful, if the iris plane is determined such that the projection of the pupil diameter onto the anterior surface may be calculated more precisely (i.e., individually for the patient's eye geometry). To this end, the apparatus and/or the corneal tomographer may include means for determining the iris plane and/or means for obtaining such information. Alternatively, such corneal regions may be arbitrarily selected by the operator.

The control unit 105 may be adapted to obtain information regarding an optical axis of the eye. The optical axis may be determined by the control unit 105, e.g. based on information provided by the tomographer or predefined by the operator. It may also be determined by the tomographer 101, and obtained by the control unit 105 from the tomographer 101. It may, however, also be determined by other means, and input to the control unit 105, e.g. by the operator. The optical axis may, e.g., be selected as an (approximate) symmetry axis of the cornea.

Similarly, the control unit 105 may be adapted to obtain information regarding a center of vision of the cornea, at which the optical axis may be centered. This may correspond to a corneal apex. The center of vision may be determined by the control unit 105, e.g. based on information provided by the tomographer or predefined by the operator. It may also be determined by the tomographer 101, e.g., based on the corneal apex or based on a light fixation reflex on the anterior corneal surface or based on the symmetry axis of the cornea, and obtained by the control unit 105 from the tomographer 101. It may, however, also be determined by other means, and input to the control unit 105, e.g. by the operator.

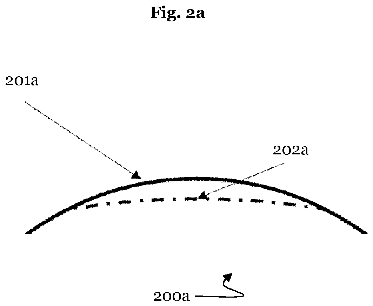

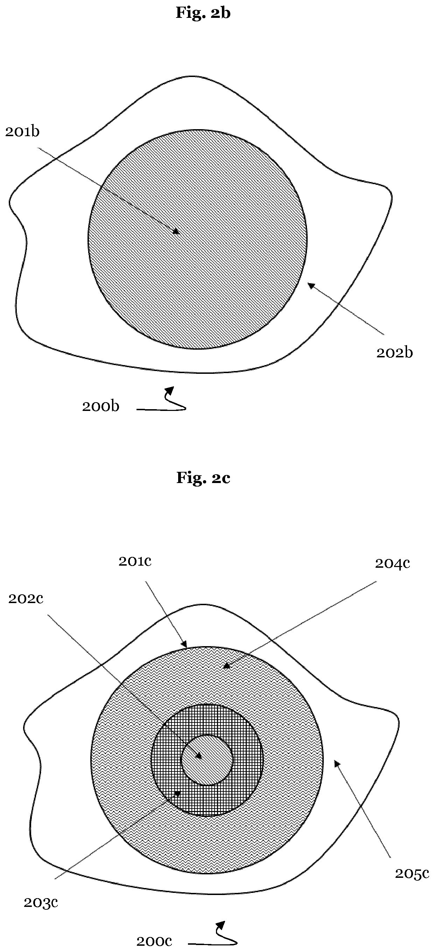

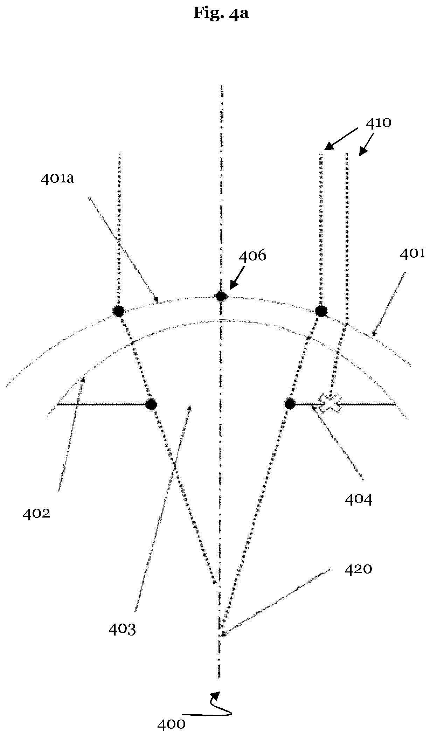

The topographic information obtained, e.g., from the corneal tomographer 101 and possibly the pupillary information collected from the pupillometer 103, the refractive aberration information obtained, e.g., from the operator 102 and possibly by the scanning laser refractometer and/or by the wave-front analyzer 104, may be obtained by the control unit 105 of the apparatus. The control unit 105 may then determine based on the obtained information a correction information. The control unit may be adapted to determine the correction information as generally described in the above section "Summary of the Invention", and as further described with respect to Figs. 2-6 below. In some examples, also further properties of the individual patient's eye may be taken into account in the determination of the correction information, e.g. topographic information regarding the retina and/or the fovea relative to the cornea. The correction information may for example be expressed in terms of a customized ablation volume of corneal tissue. For example, the correction information may relate to an ablation depth, e.g., expressed in micrometers as a function of planar x and y (or any other) coordinates of the anterior corneal surface. The topographic information of the anterior and posterior corneal surfaces may relate to similar coordinates. Further exemplary details on the determination of the correction information will be provided with reference to Figs. 4a-c, 5a-b, and Fig. 6. The control unit 105 may further be adapted to mutually control and coordinate the further elements of the apparatus.

The determined correction information may then be provided, e.g., to the excimer or solid-state laser 106, which may ablate the corneal tissue by using, e.g., a sequence of deliberately set of "laser shots". The laser 106 may comprise a coupling interface for reading the correction information from the control unit 105. After performing one or more "laser shots", at least in part based on the correction information, the resulting anterior corneal shape may be inspected, e.g., by means of a topographic (or morphological) and/or dioptrical inspection. In an example, the corneal tomographer 101 may provide updated topographic information of the anterior corneal surface after ablation. In another example, the scanning laser refractometer and/or the wave-front analyzer 104 may additionally or alternatively provide updated refractive aberration information after ablation. This updated topographic information and/or refractive aberration information may then be used to determine whether the target anterior corneal surface has been obtained. If, after the entire ablation has been performed, the desired anterior surface or refractive error elimination has not been achieved, the control unit 105 may determine a further volume for corneal tissue ablation, which may then be removed by the excimer or solid-state laser 106. Instead of applying such feedback after the entire ablation has been performed, it may also be applied in real-time, or after ablating certain portions of the entire planned ablation, for example, such that possible calibration errors of the laser may be taken into account. If the target profile of the anterior corneal surface is obtained, then the procedure for customized ablation may be stopped.

In other examples, the corneal tomographer 101 and/or the laser 106 may not necessarily be part of the apparatus.

Fig. 2a shows a schematic representation of a corneal cross-section 200a. The schematic representation depicts topographic information, i.e. a profile 201a, of the anterior corneal surface, e.g., measured by using a corneal tomographer. Further, a target profile 202a of the anterior corneal surface is depicted. The volume delimited by the actual profile 201a and the target profile 202a of the anterior corneal surface defines the volume of the corneal tissue which may be ablated, e.g., by an excimer or a solid-state laser, i.e. the target ablation volume.

Fig. 2b shows a schematic representation of a top view of a region of cornea 200b which may be analyzed and/or treated. The region comprises a monofocal zone 201b. The monofocal zone 201b may define the region of the cornea which may be treated such that the quality of vision is optimized for far distances. In an example, the monofocal zone may be determined by the treating doctor and/or by the pupillometer 103 explained with reference to Fig. 1, e.g. selected to correspond to a projection of the pupil diameter detected according to a predefined light environment. The monofocal zone 201b may additionally be surrounded by a customized connecting zone 202b. The customized connecting zone 202b connects the treated zone, i.e., the monofocal zone 201b in this example, with an untreated area of the anterior corneal surface. A perimeter of the customized connecting zone 202b may be arbitrarily chosen such that the perimeter comprises an irregular shape. The customized connecting zone 202b may be used and its perimeter may be chosen such as to minimize a risk of regression, haze, and starburst effects, and it may further facilitate healing of the cornea after treatment.

Fig. 2c shows a further schematic representation of a top view of a region of a cornea 200c. The region of the cornea comprises a multifocal zone 201c, and a customized connecting zone 205c surrounding the multifocal zone 201c. Similarly as explained with regard to Fig. 2b, the customized connecting zone 205c may connect the perimeter of the treated zone, i.e., the multifocal zone 201c in this example, with the untreated area of the anterior corneal surface. The multifocal zone 201c may be further subdivided into a near vision zone 202c, an intermediate vision zone 203c, and a far vision zone 204c. As explained with reference to the example in Fig. 2b, the multifocal zone 201c as well as the sub-zones of the multifocal zone 201c may be determined by the treating doctor and/or by the pupillometer 103 of Fig. 1. Further, the operator may determine a different refractive correction and/or different refractive error maps for the sub-zones of the multifocal zone 201c, such that a different refractive aberration information and/or a different desired focusing point may be used in each of the zones. The refractive aberration information may comprise refractive aberration information specific to one or more of the near vision zone 202c, the intermediate vision zone 203c, and the far vision zone 204c. Similarly, the desired focusing point may differ for these zones. The corresponding correction information for each zone, may then be determined based thereon, as generally outlined herein, e.g. by considering only the regions of the anterior and corresponding posterior corneal surfaces falling within the respective zones. Thus, the near vision zone 202c for vision in a near field, the intermediate vision zone 203c for vision in an intermediate field, and the far vision zone 204c for vision in a far field may be optimized individually. In other words, the correction information may comprise correction information specific to one or more of the near vision zone 202c, the intermediate vision zone 203c, and the far vision zone 204c.

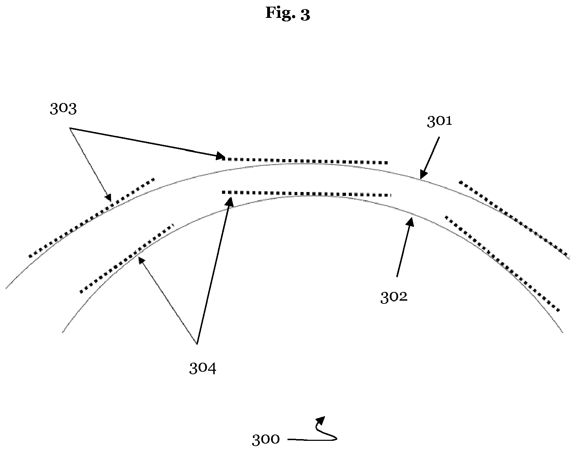

Fig. 3 shows another schematic representation of a corneal cross-section 300. The cross-section 300 shows topographic information, i.e. a profile 301, of the anterior corneal surface and topographic information, i.e. a profile 302, of the posterior corneal surface. The profiles of both the anterior and the posterior corneal surfaces may be obtained, e.g., by the corneal tomographer 101 explained with reference to Fig. 1. Further, Fig. 3 depicts slopes 303 and 304 at various points on the anterior corneal surface 301 and, respectively, the posterior corneal surface 302. The slopes 303 and 304 are relevant for the refractive behavior of the cornea (e.g., to calculate angles of refraction as determined by Snell's law), and they may also be determined by the corneal tomographer 101 or derived from information provided by the corneal tomographer 101, e.g., by calculating the respective tangent. As discussed in more detail below, the slopes 303 of the anterior corneal surface may be modified by means of laser ablation such that a target profile of the anterior corneal surface 301 may be obtained which optimizes vision in view of the refraction provided by the slopes 304 of the posterior corneal surface 302.

Fig. 4a shows another schematic representation of a corneal cross-section 400. Cross-section 400 comprises topographic information, i.e., a profile 401, of the anterior corneal surface and topographic information, i.e., a profile 402, of the posterior corneal surface. Moreover, the schematic comprises an iris plane 404, wherein the iris defines a pupil 403. Pupil 403 may correspond to a photopic pupil. An optical axis 420 may be defined, e.g., based on the visual axis of the eye, e.g. being an axis of symmetry of the cornea, e.g. selected by the operator. Moreover, a center 406 of the anterior corneal surface may defined, e.g., as the corneal apex or the light fixation reflex on the anterior corneal surface or based on the symmetry axis of the cornea or selected by the operator.

Optionally, a region of the anterior corneal surface 401a may be determined, which corresponds to a projection of the photopic pupil (i.e., assuming rays impinging onto the cornea in a manner parallel to the optical axis). Hence, that region of the anterior corneal surface 401a that is relevant to determine the desired focusing point (light rays impinging on other regions of the cornea will be blocked by the iris) may be determined. Based on the topographic information of both the anterior and posterior surfaces, and based on the generally known refractive indices of the cornea and the aqueous humour within the cornea, the rays may be traced by calculating the respective refraction angles, e.g., based on Snell's law.

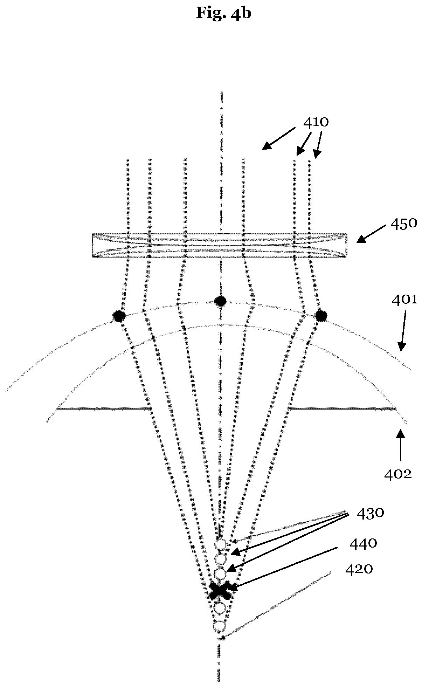

Fig. 4b shows an example for determining a desired focusing point based on topographic information for the anterior surface 401, based on topographic information for the posterior surface 402, and based on refractive aberration information 450. In this example, light rays 410 are propagating towards the cornea in a manner parallel to the optical axis 420. The light rays are refracted by optical means, e.g. mimicking a lens, that corresponds to a correction of the refractive aberration information 450. For example, if the refractive aberration information corresponds to a subjective refraction with a spherical and a cylindrical aberration with a relative axis, the optical means 450 could be a lens adapted to correct that spherical and cylindrical aberration. In case of more complex refractive aberration information, e.g. involving higher order aberrations, the optical means 450 may be defined accordingly, e.g., mimicking a more complex lens.

The light rays 410 refracted by the optical means 450 subsequently impinge on the anterior corneal surface 401, propagate through the cornea and impinge on the posterior corneal surface 402. Based on the topographic information, the refraction at these surfaces may be calculated and the rays may be traced such that their intersections 430 with the optical axis 420 within the eye may be determined. Here, the calculation may be limited to rays impinging on a region of the anterior corneal surface, e.g., region 401a determined as outlined with respect to Fig. 4a. Alternatively, the calculation may be limited to any of regions 202c, 203c or 204c as outlined with reference to Fig. 2c, e.g. if near-vision, intermediate vision or far-vision is to be analyzed separately.

From the intersections 430, a desired focusing point 440 may be determined. For example, a weighted average of all intersections may be used. In the simplest case, a center-of-mass calculation may be used, or a calculation with varying weight factors may be used, e.g. giving more weight to central rays. A decreasing weight may be given to the determined intersections according to the radial distance between the center of vision and the respective impinging light ray on the anterior corneal surface.

It is noted that in other examples, the desired focusing point may be defined differently, e.g. based on the refractive aberration 450 the operator would like to correct, based on the position of the fovea or retina of the patient's eye, or arbitrarily by the operator. The corresponding determination may be performed by the operator or a separate device, in which case the apparatus according to the present invention has a corresponding interface to obtain the desired focusing point. However, the corresponding determination may also be performed by the apparatus itself.

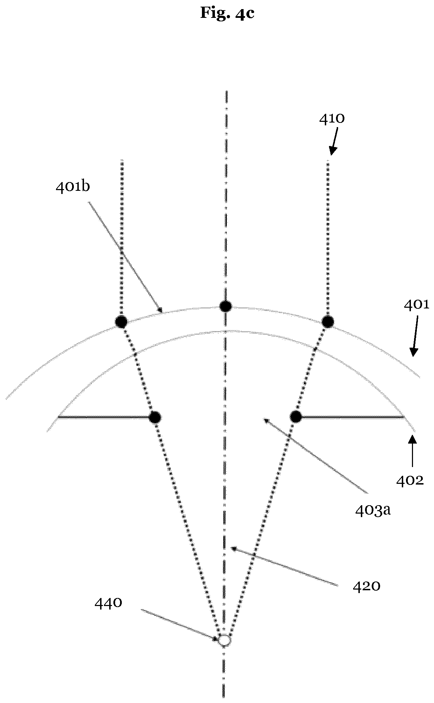

Fig. 4c shows a further step that may be implemented, in which a region 401b of the anterior corneal surface is determined, to which the subsequent calculation of the correction information and/or treatment may be limited. The region 401b may be determined based on the desired focusing point 440 and on a selected pupil diameter 403a, for example a pupil diameter detected according to a predefined light environment or a pupil diameter by which the patient's actual pupil diameter during daily life is expected to be limited with a certain likelihood. For example, a diameter may be selected such that 80%-100%, preferably about 95%, of all situations during the patient's daily life comprise a pupil diameter that does not exceed the selected diameter.

The apparatus shown in Fig. 1, specifically control unit 105, may be adapted to perform any of the steps outlined with reference to Figs. 4a-4c.

Already with respect to Figs. 4a-c, it becomes clear that taking into account topographic information regarding the posterior corneal surface of the individual patient improves the accuracy of the result, since possible irregularities of the posterior shape may be taken into account.

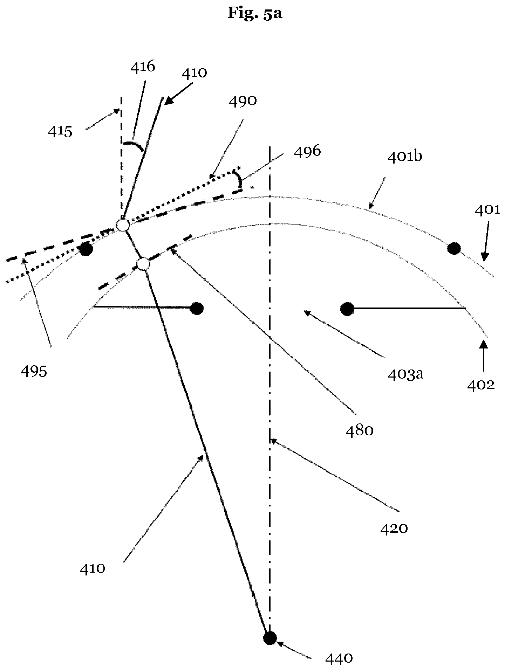

Fig. 5a shows a first example for determining correction information by reverse ray-tracing. In this example, the determination is based on a light ray 410 emerging from the desired focusing point 440. To each point or portion (e.g. of a grid, as outlined earlier) on a selected region 401b of the anterior corneal surface 401 and each corresponding point on the posterior corneal surface 402, a light ray may be traced. For each point, a correction information may be determined in the form of an angle 416 between the light ray 410, as exiting the cornea, and the direction 415 of the optical axis 420. An angle deviation between the light ray 410 as exiting from the cornea and the direction of the optical axis 420 indicates ametropia. The determination includes determining a refraction of the light ray 410 when impinging on the posterior corneal surface 402 having a local slope 480 (which may be included in or determined from the topographic information of the posterior surface 402, e.g. a profile of the posterior surface). Similarly, it includes determining a refraction of the light ray 410 impinging on the anterior corneal surface 401 (after having propagated through the cornea) having a local slope 490.

A local target slope 495 of the anterior surface 401 may then be determined such that the light beam 410 impinging on an anterior surface having such slope 495 would be refracted such that it exits the cornea in the direction 415 of the optical axis 420. Additionally or alternatively, an angle 496 between the local slope 490 and the local target slope 495 of the anterior surface 401 may be calculated. In that manner, the local target slopes 495 may be determined for each portion or point within region 401b. For example, by integrating the local target slopes 495, correction information in the form of a desired target profile 470 of the region 401b of the anterior surface 401 may be determined.

Optionally, the desired target profile 470 of the corneal anterior surface 401 within region 401b is then translated along the optical axis 420 such that a smooth transition between the target profile within region 401b and the anterior surface 401 outside region 401b may be obtained if desired. In this step, it is also ensured that all points of the target profile are located within the actual profile of the anterior corneal surface 401.

The above steps outlined with reference to Fig. 5a may then be re-iterated one or more times, wherein the topographic information of the anterior surface is replaced by the target profile of the anterior surface obtained from the respective previous iteration. Such iteration may yield a more and more refined target profile, which optimizes focusing to the desired focusing point 440. Possibly, modifications to the angles 496 are applied in each iteration to optimize focusing onto the desired focusing point 440. By iterating the process, focusing optimization onto the desired focusing point 440 may be achieved.

Additionally or alternatively to the translating of the desired target profile 470 of the corneal anterior surface 401 within region 401b along the optical axis 420 in between the iterations, such translation may be performed as a final step, i.e., after completing the iteration(s).

From an intersection between the topographic information of the anterior surface 401 and the target profile of the anterior corneal surface 202a defined as the determined target profile 470, possibly inclusive of the connecting zone 202b, an ablation volume may be determined. Correction information may thus also be provided in the form of an ablation volume.

Particularly in the calculation outlined with respect to Fig. 5a, it is beneficial to take into account topographic information regarding the posterior corneal surface. Local irregularities of the posterior shape may strongly influence the refraction at each point as well as the correction needed at each point. Taking these into account leads to an improved vision correction optimized for the individual patient, and may minimize the ablation volume.

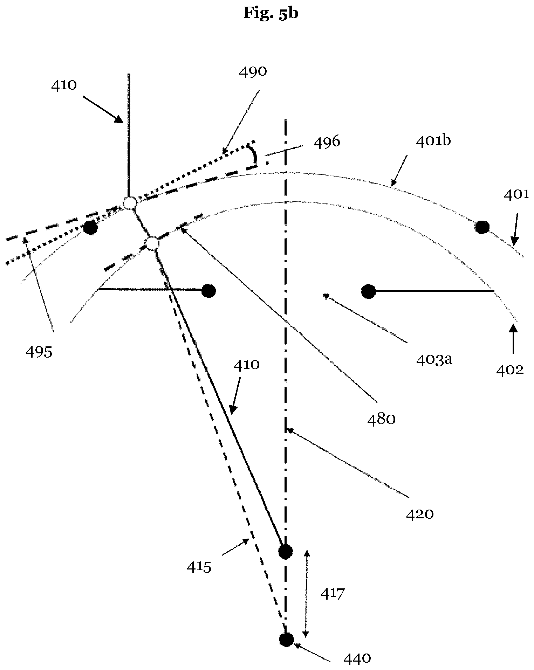

Fig. 5b shows a further example for determining correction information, which is similar to that shown in Fig. 5a. However, instead of using rays emerging from the desired focusing point 440, as outlined with reference to Fig. 5a, rays 410 are used that impinge on the selected region 401b of the cornea in a manner parallel to the optical axis 420, i.e. forward ray-tracing is used. Similarly, as outlined with reference to Fig. 5a, based on local slopes 490 and 480 of the anterior corneal surface 401 and the posterior corneal surface 402, respectively, each ray 410 may be traced until it intersects the optical axis 420 within the eye. For each ray, a position deviation 417 of that intersection from the desired focusing point 440 may be determined.

A local target slope 495 of the anterior surface 401 may be determined such that the light beam 410 is focused onto the desired focusing point 440 (or a deviation therefrom is minimized). Additionally or alternatively, an angle 496 between the local slope 490 and the local target slope 495 of the anterior surface may be determined. In that manner, the local target slopes 495 may be determined for each portion or point within a selected region 401b. For example, by integrating the local target slopes 495, a desired target profile of the anterior surface 401 may be determined, as outlined above with reference to Fig. 5a. Moreover, also the iterations and/or translation(s) along the optical axis 420 may be performed similarly also for the example outlined with reference to Fig. 5b.

Similarly as explained with reference to Fig. 5a, it is especially beneficial to take into account topographic information regarding the posterior corneal surface also for the determinations of Fig. 5b.

The determinations outlined with reference to Figs. 5a and 5b may be applied alternatively or in addition to each other, e.g. alternatingly in one or more iterations, or separately from each other, wherein a weighted average (or a simple average) of the respectively determined correction information may then be determined. The regions 401b may be selected as any of the regions outlined herein.

The apparatus shown in Fig. 1, specifically control unit 105, may be adapted to perform any of the steps outlined with reference to Figs. 5a-5b.

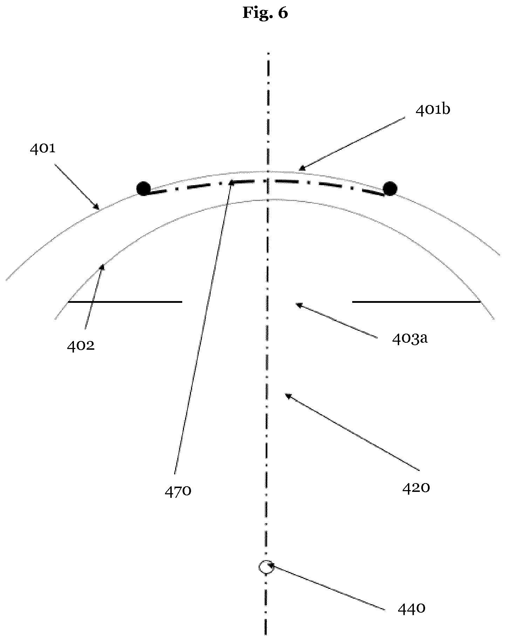

Fig. 6 shows a further exemplary cross-section, in which exemplary correction information in the form of target profile 470 of the anterior corneal surface is shown.

## Claims

1. An apparatus for visual ametropia correction, comprising:
a. means (101, 103) for obtaining topographic information of an anterior surface (401) and topographic information of a posterior surface (402) of a cornea of an eye;
b. means (102, 104) for obtaining a desired focusing point (440) within the eye, wherein the means for obtaining the desired focusing point is adapted to determine the desired focusing point based on the topographic information of the anterior surface (401) and of the posterior surface (402) of the cornea, and based on refractive aberration information (450) for the eye;
wherein the means for obtaining the desired focusing point is further adapted to determine the desired focusing point by ray tracing of a set of light rays parallel to an optical axis of the eye refracted according to the topographic information of the anterior surface (401) and of the posterior surface (402) of the cornea, and according to optical means that correspond to a correction of the refractive aberration information (450) for the eye;
c. means (105) for determining correction information (470) relating to the anterior surface of the cornea, such as to optimize focusing onto the determined desired focusing point, based on the topographic information of the anterior surface (401) and of the posterior surface (402) of the cornea.

2. The apparatus according to claim 1, wherein the correction information (470) comprises a target profile and/or a target slope of the anterior surface of the cornea.

3. The apparatus according to claim 1 or 2, wherein the means (105) for determining the correction information is adapted to perform, based on the topographic information of the anterior surface (401) and of the posterior surface (402) of the cornea, ray-tracing for at least one light ray (410) passing through the cornea and being refracted by the anterior and posterior surfaces of the cornea.

4. The apparatus according to claim 3, wherein the correction information (470) is determined at least in part such as to optimize focusing of the at least one light ray (410) onto the desired focusing point (440).

5. The apparatus according to claim 3 or 4, wherein the ray-tracing for the at least one light ray (410) relates to at least one light ray impinging on the anterior surface of the cornea in a manner parallel to an optical axis (420) of the eye.

6. The apparatus according to claim 5, wherein the correction information relating to the anterior surface of the cornea is determined at least in part such as to minimize a distance (417) between the desired focusing point and an intersection of the at least one light ray impinging on the anterior surface of the cornea with the optical axis (420) of the eye, after being refracted by the anterior and posterior corneal surfaces.

7. The apparatus according to any of claims 3-6, wherein the ray-tracing for the at least one light ray (410) relates to at least one light ray emerging from the desired focusing point (440).

8. The apparatus according to claim 7, wherein the correction information relating to the anterior surface of the cornea is determined at least in part such as to minimize an angle (416) between an optical axis of the eye (420) and the at least one light ray (410) emerging from the desired focusing point (440) as it exits the cornea, after being refracted by the posterior and anterior corneal surfaces.

9. The apparatus according to any of claims 1-8, wherein the refractive aberration information (450) comprises subjective refraction provided by the operator (102).

10. The apparatus according to any of claims 1-9, wherein the apparatus further comprises means for obtaining refractive aberration information for the eye from a scanning laser refractometer or a wave-front analyzer (104).

11. The apparatus according to any of claims 1-10, wherein the means for obtaining the topographic information of the anterior surface (401) and of the posterior surface (402) comprises a corneal tomographer (101).

12. The apparatus according to any of claims 1-11, further comprising means for correcting an aberration of the eye based on the determined correction information (470), preferably a laser (106) to remove corneal tissue.

13. The apparatus according to claim 12, further comprising means for controlling the means for correcting, preferably the laser (106), based on updated topographic information of the anterior surface of the cornea.

14. A computer program for visual ametropia correction, the computer program comprising instructions which, when the program is executed by a computer, cause the computer to:
a. obtain topographic information of an anterior surface (401) and topographic information of a posterior surface (402) of a cornea of an eye;
b. obtain a desired focusing point (440) within the eye;
c. determine correction information (470) relating to the anterior surface of the cornea, such as to optimize focusing onto the desired focusing point, based on the topographic information of the anterior surface (401) and of the posterior surface (402) of the cornea;
the computer program further including instructions for determining the desired focusing point based on the topographic information of the anterior surface (401) and of the posterior surface (402) of the cornea, and based on refractive aberration information (450) for the eye; and
wherein the instructions for determining the desired focusing point include instructions for determining the desired focusing point by ray tracing of a set of light rays parallel to an optical axis of the eye refracted according to the topographic information of the anterior surface (401) and of the posterior surface (402) of the cornea, and according to optical means that correspond to a correction of the refractive aberration information (450) for the eye.

15. A computer-implemented method for determining a visual ametropia correction, comprising the steps of:
a. obtaining topographic information of an anterior surface (401) and topographic information of a posterior surface (402) of a cornea of an eye;
b. obtaining a desired focusing point (440) within the eye, wherein the obtaining comprises determining the desired focusing point based on the topographic information of the anterior surface (401) and of the posterior surface (402) of the cornea, and based on refractive aberration information (450) for the eye;
wherein the obtaining the desired focusing point further comprises determining the desired focusing point by ray tracing of a set of light rays parallel to an optical axis of the eye refracted according to the topographic information of the anterior surface (401) and of the posterior surface (402) of the cornea, and according to optical means that correspond to a correction of the refractive aberration information (450) for the eye;
c. determining correction information (470) relating to the anterior surface of the cornea, such as to optimize focusing onto the determined desired focusing point (440), based on the topographic information of the anterior surface (401) and of the posterior surface (402) of the cornea.

## Patentansprüche

1. Vorrichtung zur visuellen Fehlsichtigkeitskorrektur, umfassend:
a. Mittel (101, 103) zum Erhalten topographischer Informationen einer vorderen Oberfläche (401) und topographischer Informationen einer hinteren Oberfläche (402) einer Hornhaut eines Auges;
b. Mittel (102, 104) zum Erhalten eines gewünschten Fokussierungspunkts (440) innerhalb des Auges, wobei das Mittel zum Erhalten des gewünschten Fokussierungspunkts angepasst ist, um den gewünschten Fokussierungspunkt basierend auf den topographischen Informationen der vorderen Oberfläche (401) und der hinteren Oberfläche (402) der Hornhaut und basierend auf Refraktionsaberrationsinformationen (450) für das Auge zu bestimmen;
wobei das Mittel zum Erhalten des gewünschten Fokussierungspunkts ferner angepasst ist, um den gewünschten Fokussierungspunkt durch Strahlverfolgung eines Satzes von Lichtstrahlen parallel zu einer optischen Achse des Auges, die gemäß den topographischen Informationen der vorderen Oberfläche (401) und der hinteren Oberfläche (402) der Hornhaut gebrochen werden, und gemäß optischen Mitteln, die einer Korrektur der Refraktionsaberrationsinformationen (450) für das Auge entsprechen, zu bestimmen;
c. Mittel (105) zum Bestimmen von Korrekturinformationen (470) in Bezug auf die vordere Oberfläche der Hornhaut, um die Fokussierung auf den bestimmten gewünschten Fokussierungspunkt basierend auf den topographischen Informationen der vorderen Oberfläche (401) und der hinteren Oberfläche (402) der Hornhaut zu optimieren.

2. Vorrichtung nach Anspruch 1, wobei die Korrekturinformationen (470) ein Zielprofil und/oder eine Zielneigung der vorderen Oberfläche der Hornhaut umfassen.

3. Vorrichtung nach Anspruch 1 oder 2, wobei das Mittel (105) zum Bestimmen der Korrekturinformationen angepasst ist, um basierend auf den topographischen Informationen der vorderen Oberfläche (401) und der hinteren Oberfläche (402) der Hornhaut Strahlverfolgung für mindestens einen Lichtstrahl (410) durchzuführen, der durch die Hornhaut hindurchgeht und durch die vordere und hintere Oberfläche der Hornhaut gebrochen wird.

4. Vorrichtung nach Anspruch 3, wobei die Korrekturinformationen (470) zumindest teilweise bestimmt werden, um die Fokussierung des mindestens einen Lichtstrahls (410) auf den gewünschten Fokussierungspunkt (440) zu optimieren.

5. Vorrichtung nach Anspruch 3 oder 4, wobei sich die Strahlverfolgung für den mindestens einen Lichtstrahl (410) auf mindestens einen Lichtstrahl bezieht, der auf die vordere Oberfläche der Hornhaut auf eine Weise parallel zu einer optischen Achse (420) des Auges auftrifft.

6. Vorrichtung nach Anspruch 5, wobei die Korrekturinformationen in Bezug auf die vordere Oberfläche der Hornhaut zumindest teilweise bestimmt werden, um einen Abstand (417) zwischen dem gewünschten Fokussierungspunkt und einem Schnittpunkt des mindestens einen Lichtstrahls, der auf die vordere Oberfläche der Hornhaut auftrifft, mit der optischen Achse (420) des Auges zu minimieren, nachdem er durch die vordere und hintere Hornhautoberfläche gebrochen wurde.

7. Vorrichtung nach einem der Ansprüche 3-6, wobei sich die Strahlverfolgung für den mindestens einen Lichtstrahl (410) auf mindestens einen Lichtstrahl bezieht, der aus dem gewünschten Fokussierungspunkt (440) austritt.

8. Vorrichtung nach Anspruch 7, wobei die Korrekturinformationen in Bezug auf die vordere Oberfläche der Hornhaut zumindest teilweise bestimmt werden, um einen Winkel (416) zwischen einer optischen Achse des Auges (420) und dem mindestens einen Lichtstrahl (410) zu minimieren, der aus dem gewünschten Fokussierungspunkt (440) austritt, wenn er aus der Hornhaut austritt, nachdem er durch die hintere und vordere Hornhautoberfläche gebrochen wurde.

9. Vorrichtung nach einem der Ansprüche 1-8, wobei die Refraktionsaberrationsinformationen (450) subjektive Refraktion umfassen, die durch den Bediener (102) bereitgestellt wird.

10. Vorrichtung nach einem der Ansprüche 1-9, wobei die Vorrichtung ferner Mittel zum Erhalten von Refraktionsaberrationsinformationen für das Auge von einem Scanning-Laserrefraktometer oder einem Wellenfrontanalysator (104) umfasst.

11. Vorrichtung nach einem der Ansprüche 1-10, wobei das Mittel zum Erhalten der topographischen Informationen der vorderen Oberfläche (401) und der hinteren Oberfläche (402) einen Hornhauttomographen (101) umfasst.

12. Vorrichtung nach einem der Ansprüche 1-11, ferner umfassend Mittel zum Korrigieren einer Aberration des Auges basierend auf den bestimmten Korrekturinformationen (470), vorzugsweise einen Laser (106) zum Entfernen von Hornhautgewebe.

13. Vorrichtung nach Anspruch 12, ferner umfassend Mittel zum Steuern des Mittels zum Korrigieren, vorzugsweise des Lasers (106), basierend auf aktualisierten topographischen Informationen der vorderen Oberfläche der Hornhaut.

14. Computerprogramm zur visuellen Fehlsichtigkeitskorrektur, wobei das Computerprogramm Anweisungen umfasst, die, wenn das Programm von einem Computer ausgeführt wird, den Computer zu Folgendem veranlassen:
a. Erhalten topographischer Informationen einer vorderen Oberfläche (401) und topographischer Informationen einer hinteren Oberfläche (402) einer Hornhaut eines Auges;
b. Erhalten eines gewünschten Fokussierungspunkts (440) innerhalb des Auges;
c. Bestimmen von Korrekturinformationen (470) in Bezug auf die vordere Oberfläche der Hornhaut, um die Fokussierung auf den gewünschten Fokussierungspunkt basierend auf den topographischen Informationen der vorderen Oberfläche (401) und der hinteren Oberfläche (402) der Hornhaut zu optimieren;
wobei das Computerprogramm ferner Anweisungen zum Bestimmen des gewünschten Fokussierungspunkts basierend auf den topographischen Informationen der vorderen Oberfläche (401) und der hinteren Oberfläche (402) der Hornhaut und basierend auf Refraktionsaberrationsinformationen (450) für das Auge umfasst; und
wobei die Anweisungen zum Bestimmen des gewünschten Fokussierungspunkts Anweisungen zum Bestimmen des gewünschten Fokussierungspunkts durch Strahlverfolgung eines Satzes von Lichtstrahlen parallel zu einer optischen Achse des Auges, die gemäß den topographischen Informationen der vorderen Oberfläche (401) und der hinteren Oberfläche (402) der Hornhaut gebrochen werden, und gemäß optischen Mitteln, die einer Korrektur der Refraktionsaberrationsinformationen (450) für das Auge entsprechen, umfassen.

15. Computerimplementiertes Verfahren zum Bestimmen einer visuellen Fehlsichtigkeitskorrektur, umfassend die folgenden Schritte:
a. Erhalten topographischer Informationen einer vorderen Oberfläche (401) und topographischer Informationen einer hinteren Oberfläche (402) einer Hornhaut eines Auges;
b. Erhalten eines gewünschten Fokussierungspunkts (440) innerhalb des Auges, wobei das Erhalten Bestimmen des gewünschten Fokussierungspunkts basierend auf den topographischen Informationen der vorderen Oberfläche (401) und der hinteren Oberfläche (402) der Hornhaut und basierend auf Refraktionsaberrationsinformationen (450) für das Auge umfasst;
wobei das Erhalten des gewünschten Fokussierungspunkts ferner Bestimmen des gewünschten Fokussierungspunkts durch Strahlverfolgung eines Satzes von Lichtstrahlen parallel zu einer optischen Achse des Auges, die gemäß den topographischen Informationen der vorderen Oberfläche (401) und der hinteren Oberfläche (402) der Hornhaut gebrochen werden, und gemäß optischen Mitteln, die einer Korrektur der Refraktionsaberrationsinformationen (450) für das Auge entsprechen, umfasst;
c. Bestimmen von Korrekturinformationen (470) in Bezug auf die vordere Oberfläche der Hornhaut, um die Fokussierung auf den bestimmten gewünschten Fokussierungspunkt (440) basierend auf den topographischen Informationen der vorderen Oberfläche (401) und der hinteren Oberfläche (402) der Hornhaut zu optimieren.

## Revendications

1. Un appareil pour la correction de l'amétropie visuelle, comprenant :
a. un moyen (101, 103) pour l'obtention d'une information topographique d'une surface antérieure (401) et d'une information topographique d'une surface postérieure (402) de la cornée d'un œil ;
b. un moyen (102, 104) pour l'obtention d'un point de focalisation souhaité (440) à l'intérieur de l'œil, le moyen pour l'obtention du point de focalisation souhaité étant apte à déterminer le point de focalisation souhaité sur la base de l'information topographique de la surface antérieure (401) et de la surface postérieure (402) de la cornée, et sur la base d'une information d'aberration de réfraction (450) pour l'œil ;
dans lequel le moyen pour l'obtention du point de focalisation souhaité est en outre apte à déterminer le point de focalisation souhaité par repérage de rayons d'un ensemble de rayons lumineux parallèles à un axe optique de l'œil réfractés, en fonction de l'information topographique de la surface antérieure (401) et de la surface postérieure (402) de la cornée, et en fonction d'un moyen optique qui correspond à une correction de l'information d'aberration de réfraction (450) pour l'œil ;
c. un moyen (105) pour la détermination d'une information de correction (470) relative à la surface antérieure de la cornée de manière à optimiser la focalisation sur le point de focalisation souhaité déterminé, sur la base de l'information topographique de la surface antérieure (401) et de la surface postérieure (402) de la cornée.

2. L'appareil selon la revendication 1, dans lequel l'information de correction (470) comprend un profil cible et/ou une pente cible de la surface antérieure de la cornée.

3. L'appareil selon la revendication 1 ou 2, dans lequel le moyen (105) pour la détermination de l'information de correction est apte à effectuer, sur la base de l'information topographique de la surface antérieure (401) et de la surface postérieure (402) de la cornée, un repérage de rayons pour au moins un rayon lumineux (410) traversant la cornée et ayant été réfracté par les surfaces antérieure et postérieure de la cornée.

4. L'appareil selon la revendication 3, dans lequel l'information de correction (470) est déterminée au moins en partie de manière à optimiser la focalisation de l'au moins un rayon lumineux (410) sur le point de focalisation souhaité (440).

5. L'appareil selon la revendication 3 ou 4, dans lequel le repérage de rayons pour l'au moins un rayon lumineux (410) concerne au moins un rayon lumineux frappant la surface antérieure de la cornée de façon parallèle à un axe optique (420) de l'œil.

6. L'appareil selon la revendication 5, dans lequel l'information de correction relative à la surface antérieure de la cornée est déterminée au moins en partie de manière à minimiser une distance (417) entre le point de focalisation souhaité et une intersection de l'au moins un rayon lumineux frappant la surface antérieure de la cornée avec la surface optique (420) de l'œil après avoir été réfracté par les surfaces cornéennes antérieure et postérieure.

7. L'appareil selon l'une des revendications 3 à 6, dans lequel le repérage de rayons pour l'au moins un rayon lumineux (410) concerne au moins un rayon lumineux issu du point de focalisation souhaité (440).

8. L'appareil selon la revendication 7, dans lequel l'information de correction relative à la surface antérieure de la cornée est déterminée au moins en partie de manière à minimiser un angle (416) entre un axe optique de l'œil (420) et l'au moins un rayon lumineux (410) issu du point de focalisation souhaité lorsqu'il quitte la cornée, après avoir été réfracté par les surfaces cornéennes postérieure et antérieure.

9. L'appareil selon l'une des revendications 1 à 8, dans lequel l'information d'aberration de réfraction (450) comprend une réfraction subjective donnée par l'opérateur (102) .

10. L'appareil selon l'une des revendications 1 à 9, dans lequel l'appareil comprend en outre un moyen pour l'obtention d'une information d'aberration de réfraction pour l'œil à partir d'un réfractomètre laser à balayage ou d'un analyseur de front d'onde (104).

11. L'appareil selon l'une des revendications 1 à 10, dans lequel le moyen pour l'obtention de l'information topographique de la surface antérieure (401) et de la surface postérieure (402) comprend un tomographe cornéen (101) .

12. L'appareil selon l'une des revendications 1 à 11, comprenant en outre un moyen pour la correction d'une aberration de l'œil sur la base de l'information de correction déterminée (470), de préférence un laser (106) pour retirer du tissu cornéen.

13. L'appareil selon la revendication 12, comprenant en outre un moyen pour le contrôle du moyen de correction, de préférence le laser (106), sur la base d'une information topographique mise à jour de la surface antérieure de la cornée.

14. Un programme informatique pour la correction de l'amétropie visuelle, le programme informatique comprenant des instructions qui, lorsque le programme est exécuté par un calculateur, font en sorte que le calculateur :
a. obtienne une information topographique d'une surface antérieure (401) et une information topographique d'une surface postérieure (402) de la cornée d'un œil ;
b. obtienne un point de focalisation souhaité (440) à l'intérieur de l'œil ;
c. détermine une information de correction (470) relative à la surface antérieure de la cornée de manière à optimiser la focalisation sur le point de focalisation souhaité déterminé, sur la base de l'information topographique de la surface antérieure (401) et de la surface postérieure (402) de la cornée ;
le programme informatique comprenant en outre des instructions pour la détermination du point de focalisation souhaité sur la base de l'information topographique de la surface antérieure (401) et de la surface postérieure (402) de la cornée, et sur la base d'une information d'aberration de réfraction (450) pour l'œil ; et
dans lequel les instructions pour la détermination du point de focalisation comprennent des instructions pour la détermination du point de focalisation souhaité par repérage de rayons d'un ensemble de rayons lumineux parallèles à un axe optique de l'œil réfractés, en fonction de l'information topographique de la surface antérieure (401) et de la surface postérieure (402) de la cornée, et en fonction d'un moyen optique qui correspond à une correction de l'information d'aberration de réfraction (450) pour l'œil.

15. Un procédé mis en œuvre par calculateur pour la détermination d'une correction de l'amétropie visuelle, comprenant :
a. l'obtention d'une information topographique d'une surface antérieure (401) et d'une information topographique d'une surface postérieure (402) de la cornée d'un œil ;
b. l'obtention d'un point de focalisation souhaité (440) à l'intérieur de l'œil, l'obtention du point de focalisation souhaité comprenant la détermination du point de focalisation souhaité sur la base de l'information topographique de la surface antérieure (401) et de la surface postérieure (402) de la cornée, et sur la base d'une information d'aberration de réfraction (450) pour l'œil ;
dans lequel l'obtention du point de focalisation souhaité comprend en outre la détermination du point de focalisation souhaité par repérage de rayons d'un ensemble de rayons lumineux parallèles à un axe optique de l'œil réfractés, en fonction de l'information topographique de la surface antérieure (401) et de la surface postérieure (402) de la cornée, et en fonction d'un moyen optique qui correspond à une correction de l'information d'aberration de réfraction (450) pour l'œil ;
c. la détermination d'une information de correction (470) relative à la surface antérieure de la cornée de manière à optimiser la focalisation sur le point de focalisation souhaité déterminé, sur la base de l'information topographique de la surface antérieure (401) et de la surface postérieure (402) de la cornée.
